# EUROPEAN PATENT SPECIFICATION

(11) **EP 0 914 818 B1**
(45) Date of publication and mention of the grant of the patent: **08.06.2005**
(21) Application number: 97927372.9
(22) Date of filing: 12.06.1997
(51) Int. Cl.: A61K 9/20, A61K 47/10, A61K 47/26, A61J 3/10

(54) **INTRAORALLY RAPIDLY DISINTEGRABLE TABLET**
IM MUNDE SCHNELLZERFALLENDE TABLETTE
COMPRIME INTRA-ORAL A DESINTEGRATION RAPIDE

(30) Priority: 14.06.1996 JP 15355396; 25.03.1997 JP 7110797
(43) Date of publication of application: 12.05.1999
(73) Proprietor: KYOWA HAKKO KOGYO CO., LTD., Chiyoda-ku, Tokyo 100 (JP)
(72) Inventor: OHTA, Motohiro, Sunto-gun, Shizuoka 411 (JP); HAYAKAWA, Eiji, Susono-shi, Shizuoka 410-11 (JP); ITO, Kunio, Sunto-gun, Shizuoka 411 (JP); TOKUNO, Sanji, Tokyo 142 (JP); MORIMOTO, Kiyoshi, Mishima-shi, Shizuoka 411 (JP); WATANABE, Yasushi, Numazu-shi, Shizuoka 410 (JP)
(74) Representative: Sajda, Wolf E., Dipl.-Phys.
(86) International application number: PCT/JP1997/002032
(87) International publication number: WO 1997/047287

(56) References cited:
- JP-A- 2 172 918
- JP-A- 49 069 819
- US-A- 3 885 026
- DATABASE WPI Section Ch, Week 198748 Derwent Publications Ltd., London, GB; Class A96, AN 1987-338131 XP002156870 & JP 62 242616 A (SANKYO CO LTD), 23 October 1987 (1987-10-23)

## Description

This invention relates to a tablet rapidly disintegrable in an oral cavity.

There are various types of oral administrative medicines: tables, capsules, granules, powders, syrups and so on. However, such oral administrative medicines have several problems as follows. As to tablets and capsules, for example, it may be hard for aged persons or children whose swallowing power is weak to swallow them. And as to granules and powders, they may cause unpleasantness in a mouth after dosage or they may erroneously happen to enter into a respiratory tract or lungs. Further, they can not be taken where there is no water because water is usually required for dosage. As for syrups, it takes trouble for measuring on dosage and it is not be expected that aged persons or children measure them accurately. On the other hand, solid medicines which can be rapidly dissolved or disintegrated in an oral cavity can be taken without measuring or water, so they may be easily taken by such aged persons or children. By the way, there have been developed several types of medicines which can be rapidly dissolved or disintegrated in an oral cavity on dosing. For instance, in JP-B-62- 50445, solid medicines which can be produced from water solution that mainly contains gelatin including an active ingredient by use of freeze drying method are disclosed. And in WO93/12769, solid medicines which can be produced by drying suspension including agar are also disclosed. However, the medicines produced by the above- mentioned prior method do not have enough hardness to be taken out by pushing from PTP packages (Press Through Pack) containing the medicines. And, they require a special pharmaceutical manufacturing technology and also require an enormous investment in plants and equipments. JP-A-5-271054 and WO93/15724 disclose production methods of tables wherein tablets composed of saccaride are produced in such a manner that saccaride mixture supplied with appropriate water is compressed at a low pressure and then dried to make solid tablets. However, such methods also require a special pharmaceutical manufacturing technology and have the fear that powder composed of the tablets may adhere to the surface of a metal mold in compression process under moistening condition. It may be therefore difficult to utilize those methods for manufacturing on an industrial scale.

EP-A-0 553 777 discloses the manufacture of tablets comprising an active ingredient and a sugar or sugar alcohol having a preferred particle size of 30 to 50 µm. The optional inclusion of a disintegrator is also disclosed. The disintegrator includes com starch, potato and other starches, carmellose calcium, carmellose sodium and polyvinyl alcohol.

The inventors of the present invention have examined an intraorally rapidly disintegrable tablet which does not require a special pharmaceutical manufacturing technology and can be simply and easily produced by a normal equipment. As a result, they have found that the compressed tablet consisting essentially of sugar alcohol or saccaride, such as D- mannitol or lactose, having an average particle diameter of not more than 30 µm as a main ingredient, an active ingredient and a disintegrant can be disintegrated in a mouth within one minute and have hardness adequate for practical use, although such tablet has been considered unable to produce for a long time.

Accordingly, the present invention relates to a tablet comprising sugar alcohol or saccharide each having an average particle diameter of not more than 30 µm, an active ingredient, and a disintegrant selected from the group consisting of crospovidone, croscarmellose sodium, and low substituted hydroxypropylcellulose, wherein the sugar alcohol or the saccharide is in the amount of 60-95% by weight of the tablet, and the disintegrant is in the amount of 1-10% by weight of the tablet.

Further the present invention relates to the production method of a tablet characterized by compressing mixture which comprises sugar alcohol or saccharide each having an average particle diameter of not more than 30 µm, an active ingredient, and a disintegrant selected from the group consisting of crospovidone, croscarmellose sodium, and low substituted hydroxypropylcellulose, wherein the sugar alcohol or the saccharide is in the amount of 60-95% by weight of the tablet, and the disintegrant is in the amount of 1-10% by weight of the tablet.

In the present invention, such as D- mannitol, solbitol, or the like, which is widely used for drugs and food, can be used as sugar alcohol, and lactose and glucose or the like, which is also widely used for drugs and food, can be used as saccaride. At least one kind of sugar alcohol or saccaride is used.

As an active ingredient, followings are used, but other ingredients for oral administration can be also used.

### <drug for central nervous system>

hyprotic, anxiolytic ··· amobarbital, alprazolam, flurazepam hydrochloride, diazepam, and so on
antiepileptic ··· sodium valproate, nitrazepam, phenytoin, and so on
NSAID / analgesic antipyretic agent ··· aspirin, acetaminophen, ibuprofen, diclofenac sodium, ethenzamide, indometacin and so on
drug for Parkinson's disease ··· levodopa, amantadine hydrochloride, trihexyphenidyl hydrochloride, piroheptine hydrochloride, and so on
psychoneurosis agent ··· etizolam, amitriptyline hydrochloride, sulpiride, and so on

### <drug for peripheral nervous system>

skeletal muscle relaxant ··· chlorphenesin carbamate, chlormezanone, and so on
autonomic nervous agent ··· valethamate bromide, tofisopam, and so on
antispasmodic ··· afloqualone, and so on

### <drug for circulatory organ>

cardiac ··· ubidecarenon, aminophylline, etilefrine hydrochloride, and so on
antiarrhythmic agent ··· atenolol, pindolol, and so on
diuretic ··· spironolactone, trichlormethiazide, furosemide, and so on
antihypertensive agent ··· todrazine hydrochloride, nicardipine hydrochloride, hydralazine hydrochloride, and so on
angiotonic ··· dihydroergotamine mesilate and so on
vasodilator ··· benidipine hydrochloride, diltiazem hydrochloride, isosorbide dinitrate, and so on
hyperlipemia ··· clinofibrate, nicomol, and so on
others ··· flunarizine hydrochloride, meclofenoxate hydrochloride, cinnarizine, and so on

### <alimesitary tract drug>

antidiarrheal drug ··· loperamide hydrochloride, dimeticone, and so on
drug for peptic ulcer ··· azulene, L- glutamine, aceglutamide aluminium, cetraxate hydrochloride, cimetidine, and so on
cholagogue ··· anetholtrithion, chenodeoxycholic acid, and so on
others ··· domperidone, trimebutine maleate, metoclopramide, cisapride and so on

### <metabolic drug>

vitamin ··· alfacarcidol, tiamine hydrochloride, cobamide, vitaroxin riboflavin butyrate, ascorbic acid, phytonadione, and so on
diabetes mellitus agent ··· glybuzole, tolbutamide, and so on

### <antiallergics>

antihistamine ··· homochlorcyclizine hydrochloride, clemastine fumarate, chlorpheniramine maleate, and so on
others ··· oxatomide, ketotifen fumarate, azelastin hydrochloride, and so on

### <antineoplastics>

antimetabolite ··· fluorouracil, tegafur, and so on

### <antibiotics>

paromomycin sulfate, amoxicillin, cefaclor, cefalexin, acetylspiramycin, minocycline hydrochloride, and so on

In the present invention, crosspovidone, crosscarmellose sodium and low substituted hydroxypropylcellulose, which are widely used for drugs and food, can be used as a disintegrant. At least one kind of disintegrant is used.

Next, a production method of a tablet according to the present invention will be described hereinafter.

The tablet of the present invention can be obtained by compressing and tableting after granulating a mixed powdered component comprising sugar alcohol or saccaride having an average particle diameter of not more than 30 µm ground by means of a hammer mill or a jet mill or the like, an active ingredient, and a disintegrant. On the other hand, the tablet of the present invention also can be obtained by compressing and tableting after granulating a mixed powdered component comprising sugar alcohol or saccaride having an average particle diameter of not more than 30 µm ground by means of a hammer mill, a jet mill or the like, an active ingredient, and a disintegrant under the presence of an easily volatile disintegrating adjuvant and thereafter the disintegrant adjuvant is volatilized.

The amount of sugar alcohol or saccaride is 60 ∼ 95%, preferably about 80 ∼ 95% per one tablet.

The amount of active ingredient is different depending on the kind and dosage amount of active ingredients, however, 0.01 ∼ 30% is preferable, and more preferably 0.01 ∼ 10% per one tablet.

The amount of disintegrant present is 1 ∼ 10% per one tablet.

Easily volatile disintegrating adjuvant is such as sublimative camphor, urethane, urea, ammonium bicarbonate, benzonic acid, or the like, however, camphor is most preferable. The amount of easily volatile disintegrating adjuvant is preferably 1 ∼ 20% and more preferably 1 ∼ 10% per one tablet.

A wet granulation method using purified water, ethanol or the like can be preferably used. In the method, for example, granulation can be executed by means of a general granulator such as a fluid-bed granulator, a rotary stirring granulator or an extruding granulator. The granulated material is dried, and mixed with a lubricant, and thereafter compressed into predetermined shape. Binder, sour agent, foaming agent, sweetening agent, flavoring agent, or colorant can be added as additive. As a lubricant, such as magnesium stearate, stearic aid, stearyl alcohol, sucrose ester of fatty acid, talc, light anhydrous silicic acid, or like can be used. Binder is, for example, hydroxypropylcellulose, polyvinylpyrrolidone, hydroxypropylmethylcellulose, partially saponificated polyvinyl alcohol methylcellulose, pullulan or the like. Sour agent is citric acid, malic acid, adipic acid, ascorbic acid, or the like. Foaming agent is sodium bicarbonate, sodium carbonate, calcium carbonate, or the like. Sweetening agent is Aspartame (TM), saccharin, glycyrrhizic acid or the like. Flavoring agent is lemon, orange, pine, mint, menthol or the like. Colorant is yellow iron sesquioxide, red iron sesquioxide, tar color or the like. The amount of lubricant is preferably 0.01 ∼ 1% and more preferably 0.01 ∼ 0.5% per one tablet.

Although a method of compression is not limited in the present invention, a rotary tablet machine, a hydraulic press machine or a single punch tableting machine which have high productivity can be more preferably used. When an easily volatile disintegrating adjuvant is used, the tablet is dried by heating after compressing process. As to lubricants, they can be excluded from the powdered mixture in the granulation process, in that occasion it may be previously spread on the surfaces of punches and dies of a tableting machine before compression process. That makes the present invention more effective. Compression pressure of a rotary tablet machine may be preferably more than 300kg.

The shape of the tablet obtained in the present invention can be pills or other shapes such as a normal R surface tablet, a sugar coated R surface tablet, a tablet with square edges, a tablet with rounded edges, or a tablet with two R surfaces, or the like.

Further, the tablet may have a divisional line.

The present invention will be concretely explained according to examples and reference examples.

### Reference Example 1

1890g of D- mannitol (Towa Kasei Co., Ltd. : average particle diameter of about 60 µm) and 100g of crosspovidone (POLYPLASDONE® XL-10 : GAF Co., Ltd.) were fed in a fluid-bed granulation dryer (Glatt Co., Ltd. : WSG- type 5), purified water was sprayed, then granulated material was obtained after granulation and drying processes. 10g of magnesium stearate was added and mixed with the granulated material, and they were compressed and tableted by a rotary tablet machine (Kikusui Seiko Co., Ltd., CLEAN PRESS COLLECT TYPE 12). Tableting conditions were as follows; tablet weight was 200mg, a metal mold was 8mm diameter flat-type, and compression pressure was varied such as 150kg, 300kg, 450kg, 600kg, and 800kg.

### Example 1

D- mannitol (Towa Kasei Co., Ltd. : average particle diameter of about 60 µm) was previously ground by a jet mill (Japan Pneumatic Co., Ltd.:type PJM-1-1.5) and the pulverized D-mannitol with average particle diameter of 20 µm was obtained. 1890g of the pulverized D-mannitol and 100g of crosspovidone (POLYPLASDONE® XL-10 : GAF Co., Ltd.) was fed in a fluid- bed granulation dryer (Glatt Co., Ltd. : WSG-type 5), purified water was sprayed, and granulated material was obtained after granulation and drying processes. 10g of magnesium stearate was added and mixed with the granulated material, and they were compressed and tableted by a rotary tablet machine (Kikusui Seiko Co., Ltd., CLEAN PRESS COLLECT TYPE 12). Tableting conditions were the same as the referenc example 1.

### Reference Example 2

100g of domperidone, antiemetic agent, 1790g of lactose (DMV Co., Ltd. : average particle diameter of about 80 µm) and 100g of crosspovidone (POLYPLASDONE® XL-10 : GAF Co., Ltd.) were fed in a fluid-bed granulation dryer (Glatt Co., Ltd. : WSG-type 5), purified water was sprayed, and granulated material was obtained after granulation and drying processes. 10g of magnesium stearate was added and mixed with the granulated material, and they were compressed and tableted by a rotary tablet machine (Kikusui Seiko Co., Ltd., : CLEAN PRESS COLLECT type 12). Tableting conditions were the same as the reference example 1.

### Example 2

Lactose (DMV Co., Ltd. : average particle diameter of about 80 µm) was previously ground by a jet mill (Japan Pneumatic Co., Ltd. : type PJM-1- 1.5) and the pulverized lactose having average particle diameter of 15 µm was obtained. 1790g of the pulverized lactose, 100g of domperidone, and 100g of crosspovidone (POLYPLASDONE® XL-10: GAF Co., Ltd.) were fed in a fluid-bed granulation dryer (Glatt Co., Ltd.: WSG-type 5), purified water was sprayed, and granulated material was obtained after granulation and drying processes. 10g of magnesium stearate was added and mixed with the granulated material, and they were compressed and tableted by a rotary tablet machine (Kikusui Seiko Co., Ltd., CLEAN PRESS COLLECT TYPE 12). Tableting conditions were the same as the reference example 1.

### Example 3

The granulated material obtained in the example 1 was tableted under the condition that tablet weight was 200mg, compression pressure was 50kg/c m² , and a little magnesium stearate was coated on a metal mold (8mm diameter flat- type) and dies of a hydraulic press machine (Riken Seiki Co., Ltd.: type P-1B)

### Example 4

140g of the pulverized D-mannitol used in the example 1, 10g of domperidone, 10g of crosspovidone (POLYPLASDONE XL-10 : GAF Co., Ltd.) and 40g of camphor were mixed in a vinyl bag and tableted under the condition that tablet weight was 200mg, a metal mold diameter was 9mm, a single punch tableting machine (Okada Seiko Co., Ltd.: N-20E type both pressure powder tableting machine) was used, and compression pressure was 1500kg/cm². The compressed tablet was dried for 10 minutes at 80 °C under vacuum condition in a vacuum dryer.

Next, the hardness and disintegrating time of the tablet of the present invention will be described using an experimental example.

### Experimental Example

The hardness and the disintegrating time of the tablet obtained by the examples 1 and 2 and reference examples 1 and 2 were measured. The hardness of the tablet was measured by a tablet destructive strength measuring instrument (Toyama Sangyo Co., Ltd. : TH- 203CP type) The disintegrating time of the tablet was measured in such a way that the tablet was placed on a No.10 wire cloth, water was dropped at a speed of 4ml/min. on the tablet, and the time till the tablet go through the wire cloth was measured. The time was determined as the disintegrating time.

The result is shown in a Table 1.

**Table 1**

| sample/compression pressure | | 150kg | 300kg | 450kg | 600kg | 800kg |
|---|---|---|---|---|---|---|
| comparison 1 | hardness | hard to be tableted | hard to be tableted | hard to be tableted | 1.9kgf | 2.3kgf |
| | disintegration | - | - | - | 20sec. | 22sec. |
| embodiment 1 | hardness | 1.9kgf | 3.9kgf | 5.1kgf | 6.2kgf | 7.3kgf |
| | disintegration | 10sec. | 15sec. | 16sec. | 19sec. | 27sec. |
| comparison 2 | hardness | hard to be tableted | hard to be tableted | hard to be tableted | 1.5kgf | 2.1kgf |
| | disintegration | - | - | - | 18sec. | 21sec. |
| embodiment 2 | hardness | 1.6kgf | 4.0kgf | 4.9kgf | 5.8kgf | 6.5kgf |
| | disintegration | 10sec. | 16sec. | 20sec. | 25sec. | 29sec. |

In reference examples 1 and 2, tableting was hard till 450kg compression pressure and tableting was made possible at around 600kg. However, the hardness of the tablet was not enough. In examples 1 and 2, enough tablet hardness could be obtained at more than 300kg compression pressure and the disintegrating time was very fast. When the tablet produced at 450kg compression pressure according to the example 1 was dosed, the tablet was disintegrated within 10 seconds in an oral cavity.

The tablets obtained by the examples 3 and 4 were also measured of its hardness and disintegrating time in the same way. The tablets obtained by the example 3 had enough hardness of about 6.5kgf and its disintegrating time was about 10 seconds. The hardness of the tablet of the example 4 was about 4kgf and its disintegrating time was about 2 seconds, which was very fast.

### Industrial Applicability

According to the present invention, a tablet rapidly disintegrable in an oral cavity can be provided.

## Claims

1. A tablet comprising:
sugar alcohol or saccharide each having an average particle diameter of not more than 30 µm,
an active ingredient, and
a disintegrant selected from the group consisting of crospovidone, croscarmellose sodium, and low substituted hydroxypropylcellulose,
wherein the sugar alcohol or the saccharide is in the amount of 60-95% by weight of the tablet, and
the disintegrant is in the amount of 1-10% by weight of the tablet.

2. The tablet as set forth in claim 1,
wherein the sugar alcohol or the saccharide is in the amount of 80-95% by weight of the tablet.

3. The tablet as set forth in claim 1 or 2,
wherein the sugar alcohol is D-mannitol.

4. The tablet as set forth in claim 1 or 2, wherein the saccharide is lactose.

5. The tablet as set forth in any one of claims 1 to 4,
wherein the active ingredient is in the amount of 0.01-30% by weight of the tablet.

6. A production method of a tablet **characterized by** compressing mixture which comprises
sugar alcohol or saccharide each having an average particle diameter of not more than 30 µm,
an active ingredient, and
a disintegrant selected from the group consisting of crospovidone, croscarmellose sodium, and low substituted hydroxypropylcellulose,
wherein the sugar alcohol or the saccharide is in the amount of 60-95% by weight of the tablet, and
the disintegrant is in the amount of 1-10% by weight of the tablet.

7. The production method as set forth in claim 6,
wherein the sugar alcohol or the saccharide is in the amount of 80-95% by weight of the tablet.

8. The production method as set forth in claim 6 or 7,
wherein the sugar alcohol is D-mannitol.

9. The production method as set forth in claim 6 or 7,
wherein the saccharide is lactose.

10. The production method as set forth in any one of claims 6 to 9,
wherein the active ingredient is in the amount of 0.01-30% by weight of the tablet.

## Patentansprüche

1. Tablette, die folgendes aufweist:
- Zuckeralkohol oder Saccharid mit jeweils einem mittleren Partikeldurchmesser von nicht mehr als 30 µm,
- einen Wirkstoff und
- ein Sprengmittel, das aus der Gruppe ausgewählt ist, die aus Crospovidon, Croscarmellose-Natrium und niedrig substituierter Hydroxypropylcellulose besteht,
wobei der Zuckeralkohol oder das Saccharid in einer Menge von 60 bis 95 Gew.-% der Tablette vorliegt und
wobei das Sprengmittel in einer Menge von 1 bis 10 Gew.-% der Tablette vorliegt.

2. Tablette nach Anspruch 1,
wobei der Zuckeralkohol oder das Saccharid in einer Menge von 80 bis 95 Gew.-% der Tablette vorliegt.

3. Tablette nach Anspruch 1 oder 2,
wobei der Zuckeralkohol D-Manitol ist.

4. Tablette nach Anspruch 1 oder 2,
wobei das Saccharid Lactose ist.

5. Tablette nach einem der Ansprüche 1 bis 4,
wobei der Wirkstoff in einer Menge von 0,01 bis 30 Gew.-% der Tablette vorliegt.

6. Verfahren zum Herstellen einer Tablette,
**gekennzeichnet durch** das Pressen eines Gemischs, das folgendes aufweist:
- Zuckeralkohol oder Saccharid mit jeweils einem mittleren Partikeldurchmesser von nicht mehr als 30 µm,
- einen Wirkstoff und
- ein Sprengmittel, das aus der Gruppe ausgewählt ist, die aus Crospovidon, Croscarmellose-Natrium und niedrig substituierter Hydroxypropylcellulose besteht,
wobei der Zuckeralkohol oder das Saccharid in einer Menge von 60 bis 95 Gew.-% der Tablette vorliegt und
wobei das Sprengmittel in einer Menge von 1 bis 10 Gew.-% der Tablette vorliegt.

7. Herstellungsverfahren nach Anspruch 6,
wobei der Zuckeralkohol oder das Saccharid in einer Menge von 80 bis 95 Gew.-% der Tablette vorliegt.

8. Herstellungsverfahren nach Anspruch 6 oder 7,
wobei der Zuckeralkohol D-Manitol ist.

9. Herstellungsverfahren nach Anspruch 6 oder 7,
wobei das Saccharid Lactose ist.

10. Herstellungsverfahren nach einem der Ansprüche 6 bis 9,
wobei der Wirkstoff in einer Menge von 0,01 bis 30 Gew.-% der Tablette vorliegt.

## Revendications

1. Comprimé comprenant :
un alcool de sucre ou un saccharide ayant chacun une granulométrie moyenne non supérieure à 30 µm,
un ingrédient actif, et
un désintégrant choisi dans le groupe constitué par la crospovidone, la croscarmellose sodique, et l'hydroxypropylcellulose faiblement substituée,
dans lequel l'alcool de sucre ou le saccharide est présent en une quantité de 60 à 95 % en poids du comprimé, et
le désintégrant est présent en une quantité de 1 à 10 % en poids du comprimé.

2. Comprimé selon la revendication 1, dans lequel l'alcool de sucre ou le saccharide est présent en une quantité de 80 à 95 % en poids du comprimé.

3. Comprimé selon la revendication 1 ou 2, dans lequel l'alcool de sucre est le D-mannitol.

4. Comprimé selon la revendication 1 ou 2, dans lequel le saccharide est le lactose.

5. Comprimé selon l'une quelconque des revendications 1 à 4, dans lequel l'ingrédient actif est présent en une quantité de 0,01 à 30 % en poids du comprimé.

6. Procédé de production d'un comprimé, **caractérisé par** la compression d'un mélange qui comprend :
un alcool de sucre ou un saccharide ayant chacun une granulométrie moyenne non supérieure à 30 µm,
un ingrédient actif, et
un désintégrant choisi dans le groupe constitué par la crospovidone, la croscarmellose sodique, et l'hydroxypropylcellulose faiblement substituée,
dans lequel l'alcool de sucre ou le saccharide est présent en une quantité de 60 à 95 % en poids du comprimé, et
le désintégrant est présent en une quantité de 1 à 10 % en poids du comprimé.

7. Procédé de production selon la revendication 6, dans lequel l'alcool de sucre ou le saccharide est présent en une quantité de 80 à 95 % en poids du comprimé.

8. Procédé de production selon la revendication 6 ou 7, dans lequel l'alcool de sucre est le D-mannitol.

9. Procédé de production selon la revendication 6 ou 7, dans lequel le saccharide est le lactose.

10. Procédé de production selon l'une quelconque des revendications 6 à 9, dans lequel l'ingrédient actif est présent en une quantité de 0,01 à 30 % en poids du comprimé.
